# EUROPÄISCHE PATENTSCHRIFT

(11) **EP 0 645 997 B1**
(45) Veröffentlichungstag und Bekanntmachung des Hinweises auf die Patenterteilung: **20.12.1995**
(21) Anmeldenummer: 93912639.7
(22) Anmeldetag: 24.06.1993
(51) Int. Cl.: A61K 7/00

(54) **PHOSPHOLIPIDE UND FLUORCARBONE ENTHALTENDES KOSMETIKUM**
COSMETIC CONTAINING PHOSPHOLIPIDS AND FLUOROCARBON COMPOUNDS
COSMETIQUE CONTENANT DES PHOSPHOLIPIDES ET DES COMPOSES FLUOROCARBONES

(30) Priorität: 26.06.1992 DE 4221255
(43) Veröffentlichungstag der Anmeldung: 05.04.1995
(73) Patentinhaber: LANCASTER GROUP AG, 67059 Ludwigshafen (DE)
(72) Erfinder: GROSS, Udo, D-13059 Berlin (DE); ZASTROW, Leonhard, MC-98000 Monaco (MC); RÖDING, Joachim, D-65207 Wiesbaden (DE); STANZL, Klaus, White Plains, N.Y. 10605 (US)
(74) Vertreter: Walter, Wolf-Jürgen
(86) Internationale Anmeldenummer: DE9300575
(87) Internationale Veröffentlichungsnummer: WO9400098

(56) Entgegenhaltungen:
- WO-A-89/08459
- WO-A-91/00110
- WO-A-92/06676
- DE-A- 4 127 442

## Beschreibung

Die Erfindung betrifft ein Phospholipide und Fluorcarbone enthaltendes Kosmetikum mit einer die Sauerstoffversorgung der Haut verbessernden Wirksamkeit.

Es ist bekannt, partikuläre Strukturen in Form wäßriger Phospholipid-Liposomen als kosmetische Präparate einzusetzen. Den aus natürlichen Phospholipiden z.B. Sojalecithin hergestellten Liposomen mit einer der Zellmembranstruktur entsprechenden lamellaren Bilayerstruktur werden strukturerneuernde Effekte und eine Verbesserung der Widerstandsfähigkeit der Haut zugeschrieben. Die Liposome durchdringen das stratum corneum und setzen sich an geschwächten Stellen der Epidermis fest und verbessern die Zwischenzellstruktur.

Eine Erhöhung der Wirksamkeit von Liposomen wird erreicht durch die Verkapselung von Wirkstoffen und die Herstellung liposomaler Kosmetika. Die DE-A-3242385 (L'OREAL) schützt eine liposomale Zusammensetzung, die in der Liposomen-Phase Polypeptidextrakte, Pflanzenextrakte (Almondermin) und UV-Lichtschutzfilter enthält.

Die Firma Dior vertreibt das Gesichtsgel "Capture", das 5 % Thymusextrakt, 1 % Collagen und Elastinpeptide sowie 0,1 % Hyaluronsäure in Liposomen von 100 nm Durchmesser aus Sojalecithin enthält. Die Anwendung erfolgt mittels eines Pumpdispersers.

Zur verbesserten Versorgung der Haut mit Sauerstoff wurde bereits vorgeschlagen, Peroxide wie Wasserstoffperoxid zu verwenden, um über den gebildeten naszierenden Sauerstoff den Zellmetabolismus der Haut zu stimulieren. Die beträchtlichen Nebenwirkungen wie die Hautreizungen standen jedoch einer Anwendung entgegen. In der DE-A-2534315 wurde eine O₂-enthaltende kosmetologische Formulierung beansprucht, die sich aus einem O₂-gesättigten gasförmigen Fluorcarbon und einem Tensid in wäßriger Phase in einem Aerosolbehälter zusammengesetzt. Von Borgarello (EP-A-296661) wurde ein isotropes Einphasensystem für den kosmetischen Sektor entwickelt, wobei halogenierte Verbindungen als Sauerstoffträger wirken sollen. Eine typische Zusammensetzung besteht aus 34 % einer Mischung von Perchlor-1-butyl-tetrahydrofuran und Polyfluor-1-propyltetrahydrofuran, 7 % iso-Propanol, 49 % Wasser und 10 % Emulgator. Als Emulgatoren werden sehr stark grenzflächenaktive Fluortenside z.B. vom Perfluoralkansulfonsäureamid-Typ verwendet, die bei i.p. Anwendung in der Haus als extrem toxisch bekannt sind (LD₅₀ 0,1 bis 0,2 g/kg) und auch auf der Haut irritierend wirken. Weitere Lösungsmöglichkeiten haben zum Inhalt die Verwendung eines Hämolymphextraktes von Schalentieren oder eines Extraktes von Proteinen und Proteiden aus Rindermilz. Eine überzeugende und physikalisch nachweisbare Tonisierung und Belebung der Hautoberfläche ist mit den erwähnten Präparaten und Methoden nicht zu erreichen.

Die WO-A-89 08 459 beschreibt eine Perfluorcarbonemulsion mit Phospholipidvesikeln als Blutersatzstoff, bei der die Phospholipidmonomeren polymerisiert werden. In der WO-A-91 00110 werden Fluorcarbonemulsionen mit Phospholipiden offenbart, bei denen das Phospholipid gesättigte Kohlenstoffbindungen hat. Aus der WO-A-9206676 sind Öl-gefüllte wenig-lamellare Vesikel aus Phospholipiden bekennt, deren Struktur der üblichen Vesikelstruktur entspricht
Der Erfindung liegt die Aufgabe zugrunde, mit Hilfe einer kosmetischen Zusammensetzung mit einem Gehalt an Phospholipiden die Sauerstoffversorgung der Haut so zu verbessern, daß ein nachweisbarer Effekt erzielt wird.

Erfindungsgemäß besteht das Phospholipide enthaltende Kosmetikum aus asymmetrischen lamellaren Aggregaten, die aus Phospholipiden und mit Sauerstoff beladenem Fluorcarbon oder Fluorcarbongemisch bestehen, wobei der Anteil an Fluorcarbon im Bereich von 0,2 bis 100 % w/v (w/v = Gewicht/Volumen) liegt, in einem für die kosmetische Anwendung geeigneten Trägerstoff.

Es können eine Vielzahl von Fluorcarbonen eingesetzt werden, z.B. aliphatische geradkettige und verzweigte Fluoralkane, mono- oder bicyclische und gegebenenfalls fluoralkylsubstituierte Fluorcycloalkane, perfluorierte aliphatische oder bicyclische Amine, Bis-(perfluoralkyl)-Ethene, Perfluorpolyether oder deren Gemische. Besonders bevorzugt sind solche Fluorcarbone wie Perfluordecalin, F-Butyltetrahydrofuran, Perfluortributylamin, Perfluoroctylbromid, Bis-Fluor(Butyl)ethen oder Bis-Fluor(hexyl)ethen oder C₆-C₉-Perfluoralkane. Dabei liegt der Anteil an Fluorcarbonen im Bereich von 20 bis 100 % w/v, vorzugsweise im Bereich von 40 bis 100 %. Ein besonders bevorzugter Bereich ist der von 70 bis 100 % w/v.

Unter dem hier verwendeten Begriff "Fluorcarbone" werden perfluorierte oder hochfluorierte Kohlenstoffverbindungen oder Gemische verstanden, die in der Lage sind, Gase wie O₂ und CO₂ zu transportieren. Hochfluorierte Kohlenwasserstoffverbindungen sind im Sinne dieser Erfindung solche, bei denen die meisten Wasserstoffatome durch Fluoratome ersetzt sind, so daß bei weiterem Ersatz nicht notwendigerweise die Fähigkeit zum Gas-transport erhöht wird. Dies wird meist dann erreicht, wenn etwa bis zu 90 % der Wasserstoffatome durch Fluoratome ersetzt sind. Bevorzugt im Sinne der vorliegenden Erfindung sind Fluorcarbone, bei denen wenigstens 95 % der Wasserstoffatome ersetzt sind, bevorzugter 98 % und am bevorzugtesten 100 %.

Als Phospholipide werden erfindungsgemäß natürliche Phospholipide wie Sojalecithin und Eilecithin sowie synthetische Phospholipide eingesetzt. Bei diesen Phospholipiden liegt der Gehalt an Phosphatidylcholin erfindungsgemäß im Bereich von 30 bis 99 % und insbesondere 70 bis 90 %.

Neben Phosphatidylcholin können auch Lysolecithine im Konzentrationsbereich von 0,1 bis 10 Gew.-% und/oder geladene Phospholipide wie Phosphatidylethanolamin, n-Acetylphosphatidylethanolamin oder Phosphatidsäure im Konzentrationsbereich 0,1 bis 30 Gew.-% vorhanden sein.

Im Unterschied zu den bekannten wäßrigen Liposomen (Vesikel) tragen die erfindungsgemäßen Phospholipid-stabilisierten Aggregate in ihrem Kern hydrophobe Fluorcarbone, die zum Transport von Sauerstoff befähigt sind. Ihre grenzflächenchemische Stabilisierung erfolgt primär durch eine Monolayer mit inverser Anordnung und gegebenenfalls ein sich daran anschließender Aufbau von Bilayer-Schichten. Wegen der Besonderheit ihrer strukturellen Anordnung werden diese neuartigen Aggregate als asymmetrische lamellare Sauerstoff-Carrier bezeichnet. Ihre außergewöhnliche kolloidchemische Stabilität ist vermutlich auf die lamellare Struktur und auf die Oberflächenladung der Aggregate zurückzuführen. Letztere ist auf die Auswahl geeigneter Phospholipide beziehungsweise deren Mischungen natürlicher wie auch synthetischer Provenienz zurückzuführen. In erster Linie sind für eine vorteilhafte Wirkung in diesem Sinne Phospholipide, insbesondere Phosphatidylcholin im genannten Konzentrationsbereich von 10 bis 99 % in Verbindung mit Lysolecithinen der Konzentration von 0,1 bis 10 % und/oder geladenen Phospholipiden im Konzentrationsbereich 0,1 bis 30 Gew.-% verantwortlich. Die angesprochene Wirkung der Phospholipide wird durch entsprechende negative Zeta-Potentiale und durch die Messung von Ladungsdichten (bei Titration mit einem kationischen Polyelektrolyten) verifiziert.

Der Vorzug der erfindungsgemäßen Phospholipiddispersionen besteht darin, daß durch eine zusätzliche über das Fluorcarbon vermittelte Sauerstoffversorgung die Durchblutung und damit die Stoffwechselprozesse in der epidermalen Schicht gefördert und der allgemeine Status der Hautatmung aktiviert werden. Mit der Erhöhung der Zellrespiration wird das natürliche Hautabwehrpotential gestärkt und die Eliminierung von Hauttoxinen gefördert. Darüber hinaus kommen durch die Anwendung des Kosmetikums in einer phospholipidstabilisierten Form wegen der wassertragenden lamellaren Schichtstrukturen die feuchtigkeitsspendende Wirkung und damit verbundene hautglättende Eigenschaften zum Tragen.

Im Gegensatz zu den eingangs erwähnten bekannten Präparaten zeigen die erfindungsgemäßen Zusammensetzungen, daß die chemisch inerten Fluorcarbone auf Grund ihres außergewöhnlich hohen Sauerstofflösevermögens bei topischer Anwendung in Form von asymmetrischen lamellaren Aggregaten die Haut vorteilhaft und dosiert mit Sauerstoff versorgen können. Das Eindringen der asymmetrischen lamellaren Aggregate konnte erstmals als eine Bestätigung des erfindungsgemäßen Effektes mit einer markierten Phospholipid-Dispersion an physiologisch intakter isolierter Haut durch ein spektroskopisches Verfahren bestätigt werden.

Die Anwendung als Kosmetikum ist nicht auf die Gesichtspartien des Menschen beschränkt, sondern betrifft alle epidermalen Bereiche des Körpers, eingeschlossen von Cellulitis betroffenes Fettgewebe mit mangelnder Durchblutung sowie den Bereich der Kopfhaut, dabei insbesondere die Haarzellen. Die topische Anwendung von Fluorcarbon enthaltenden Phospholipid-stabilisierten Aggregaten auf der Haut ist bisher nicht bekannt. Fluorcarbone selbst sind chemisch und biologisch inerte organische Flüssigkeiten mit einem hohen Sauerstofflösevermögen. Wegen dieser Eigenschaften wurden sie als Gasträger in Blutersatzemulsionen vorgeschlagen und auch am Menschen zur Anwendung gebracht (K.C. Lowe: Blood substitutes, Ellis-Horwood, Chichester, GB., 1988). Ebenso sind die natürlich vorkommenden Phospholipide wie Soja- oder Eilecithin toxikologisch unbedenklich und darüber hinaus als hautfreundlich und hautpflegend bekannt.

Die Fluorcarbone können entsprechend dem spezifischen Anwendungszweck nach O₂-Löslichkeit, Partialdampfdruck und Lipidlöslichkeit ausgewählt werden. Die kritische Löslichkeitstemperatur der Fluorcarbone (CST) in n-Hexan korreliert mit ihrer Löslichkeit in Lipiden, z.B. Zellmembranen, und stellt somit ein Maß für die Geschwindigkeit der Abgabe durch die Haut dar. So werden z.B. Perfluordecalin und Perfluoroctylbromid mit kleinen CST-Werten relativ schnell abgegeben, während andererseits F-Tributylamin mit einem hohen CST-Wert von 59 °C auch eine hohe Halbwertszeit der Abgabe aufweist. Es wurde gefunden, daß sich Fluorcarbone beim Mischen ideal verhalten und ihre CST-Werte linear von der Zusammensetzung abhängen. Damit gelingt es durch Mischen verschiedener Fluorcarbone, definierte CST-Werte einzustellen, die durch individuelle Verbindungen oft nicht realisierbar sind. Diese Erkenntnis bietet die Möglichkeit, Fluorcarbonmischungen gezielt einzusetzen, um die Penetrationsgeschwindigkeit in die Haut und ihre Verweilzeit in positiver Weise zu beeinflussen.

Die Erfindung betrifft auch ein Verfahren zur Herstellung eines Phospholipide enthaltenden Kosmetikums, das darin besteht, daß Phospholipide emulgiert werden mit einem Fluorcarbon oder einem Fluorcarbongemisch, welches mit Sauerstoff beladen ist, wobei der Anteil an Fluorcarbon im Bereich von 0,2 bis 100 % w/v liegt, und die dabei erhaltenen asymmetrischen lamellaren Aggregate mit einer mittleren Teilchengröße von 50 bis 3000 nm, insbesondere von 50 bis 1000 nm in einen für die kosmetische Anwendung geeigneten Träger eingearbeitet werden. Dabei erfolgt eine Voremulgierung der Rohdispersion durch Zugabe des Fluorcarbons zu einer wäßrigen Phospholipidlösung bei einer Temperatur entsprechend den eingesetzten Ausgangsstoffen. Die Voremulgierung erfolgt zweckdienlich bei höheren Umdrehungszahlen z.B. 12 000 bis 15 000 U/min. Die eigentliche Homogenisierung erfolgt dann mit einem Hochdruckhomogenisator. Die Durchmesser der Aggregate liegen in der Größenordnung von 50 bis 3000 nm, vorzugsweise 140 bis 320 nm. Die Teilchengrößenverteilungen können durch Zentrifugieren vereinheitlicht beziehungsweise getrennt werden. Eine Hitzesterilisierung im Autoklaven ist ohne eine Beeinflussung der Teilchengrößen möglich. Zur Vermeidung von Autoxidationsprozessen im ungesättigten Fettsäurerest nativer Lipide können Antioxidantien, z.B. α-Tocopherol zugesetzt werden.

Die verfahrensgemäß eingesetzte Lipidfraktion enthält erfindungsgemäß Phosphatidylcholin in einer Menge von 30-99 % und insbesondere 70 bis 90 %.

Die Einarbeitung der asymmetrischen lamellaren Aggregate als wirksame Substanz in Salben, Cremes, Lotionen und anderen wäßrigen oder alkoholischen kosmetischen Formulierungen erfolgt in Abhängigkeit vom Anwendungszweck, wobei der Fluorcarbongehalt und damit die O₂-Verfügbarkeit in breiten Grenzen variiert werden kann. Die Aggregate können vor der Einarbeitung in alle kosmetischen Systeme wie z.B. Gele, Pasten, Puder, Salben, Cremes, Lotionen und Wässer beziehungsweise alkoholische Auszüge mit gasförmigem Sauerstoff partiell beladen beziehungsweise gesättigt werden. Bereits die Sättigung mit dem Sauerstoff der atmosphärischen Luft durch die üblicherweise stattfindende Gleichgewichtseinstellung entsprechend dem Henry'schen Gesetz bietet eine höhere Sauerstoffkapazität als alle vergleichbaren bekannten Systeme.

Der Gehalt an asymmetrischen lamellaren Phospholipid-Aggregate in den kosmetischen Zubereitungen kann erfindungsgemäß im Bereich von 0,05 bis 80 Gew.-% liegen, vorzugsweise im Bereich von 0,05 bis 60 % und insbesondere im Bereich von 1 bis 50 Gew.-%. Besonders hervorzuheben ist, daß die erfindungsgemäßen asymmetrischen lamellaren Phospholipid-Aggregate nach Verarbeitung in den kosmetischen Zubereitungen unbeeinflußt durch die Begleitstoffe vorliegen, was für ihre besondere Stabilität spricht.

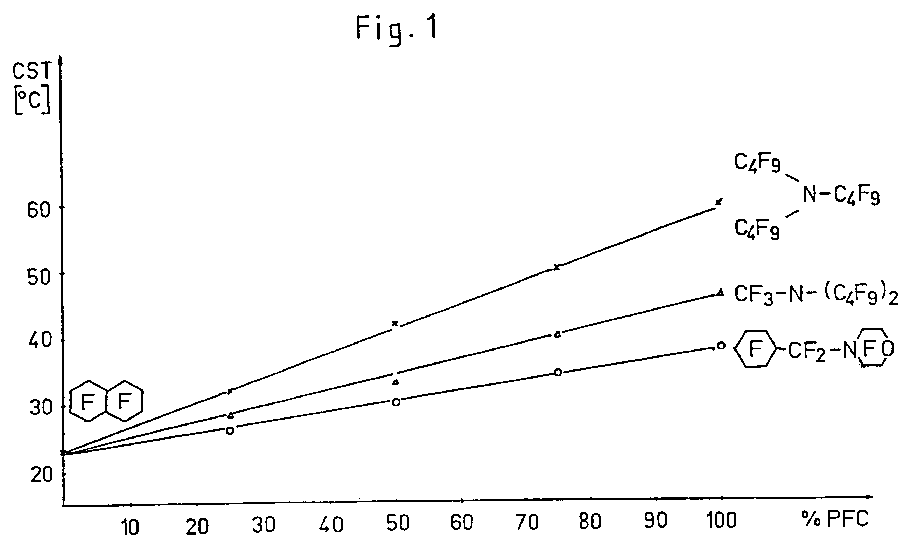

Die Erfindung soll nachstehend durch Beispiele näher erläutert werden. In der dazugehörigen Zeichnung bedeuten
Fig.1 Diagramm der kritischen Löslichkeitstemperaturen (CST) von Perfluorcarbongemischen in n-Hexan mit Perfluordecalin als Ausgangspunkt

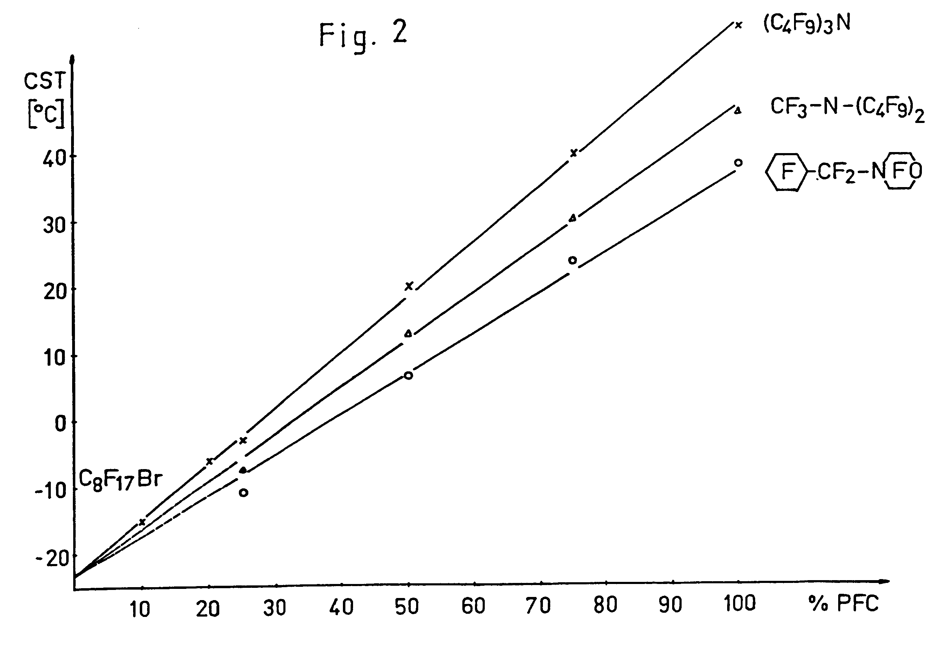

Fig.2 Diagramm der kritischen Löslichkeitstemperaturen von Perfluorcarbongemischen in n-Hexan mit F-Octylbromid als Ausgangspunkt.

In Tabelle 1 sind einige ausgewählte Fluorcarbone und ihre O₂-Löslichkeit, ihr Dampfdruck und die kritische Löslichkeitstemperatur dargestellt. Ausgehend von diesen Werten können für Gemische von Fluorcarbonen die gewünschten Charakteristika bei der Penetrierung der Haut mit Hilfe einer kosmetischen Zusammensetzung ausgewählt werden.

**Tabelle 1**

| Fluorcarbon | O₂-Löslichkeit [ml O₂/100 ml FC] | Dampfdruck P₃₇ _{°C} [mm Hg] | CST [°C] |
|---|---|---|---|
| Perfluoroctylbromid | 50 | 14 | -24,5 |
| Perfluordecalin | 40 | 12,5 | 22 |
| Bis-F(Butyl)ethen | 50 | 12,6 | 22,5 |
| F-cyclohexylmethylmorpholin | 42 | 4 | 38,5 |
| F-Tripropylamin | 45 | 18,5 | 43 |
| F-Dihexylether | 45 | 2 | 59 |
| F-Tributylamin | 40 | 1 | 59 |
| Perfluordecalin-F-Tributyl-amin 1/1 | 40 | 7 | 42 |
| Perfluorbutyltetrahydrofuran | 52 | 51 | 29 |
| F-methylcyclohexan | 57 | 180 | 8,2 |
| F-Hexan | 58 | 414 | 20 |

### Beispiel 1

50 ml einer 10%igen wäßrigen Phospholipidlösung (Sojalecithin, 80 % Phosphatidylcholin (PC)) werden gemeinsam mit 80 g eines hochreinen, keine H-Atome enthaltenden Fluorcarbongemisches (90 % Perfluordecalin, 10 % F-Dibutylmethylamin, kritische Löslichkeitstemperatur 26 °C) mit einem Ultraschalldesintegrator unter Eiskühlung homogenisiert bis die Teilchen einen mittleren Durchmesser von 244 nm aufweisen. Aus ³¹P-NMR-Messungen ist anhand der typischen Signalbreite wie auch aus elektronenmikroskopischen Aufnahmen die multilamellare Struktur der Aggregate aus Fluorcarbon und Phospholipid zu erkennen.

Die Aggregatdispersion läßt sich unproblematisch und ohne Beeinflussung ihrer Stabilität mit geeigneten Alkoholen zum Zweck der Sterilisierung mischen. Ein Zusatz von 30 ml Ethanol erzeugt Keimfreiheit, wobei die resultierende Dispersion folgende Zusammensetzung aufweist:
62 % w/v Fluorcarbone; 9,7 % Phospholipide; 19 % Ethanol
Das Zeta-Potential von minus 61 mV belegt eine durch die Phospholipide erzeugte negative Oberflächenladung mit einer elektrostatischen Stabilisierung der Dispersion. Nach Sättigung mit gasförmigem Sauerstoff wird die Dispersion in eine mit den asymmetrischen lamellaren Aggregaten verträgliche, nicht wechselwirkende Salbengrundlage eingearbeitet. Das dabei erhaltene Kosmetikum hat folgende Zusammensetzung:
20 ml Phospholipdispersion
(5 g Fluorcarbon, 2,2 g Phospholipid)
65 ml wäßrige Phase
(Polyacrylgel, Glycerol, Polyethylenglycole, Methylparaben)
15 ml ölige Phase
(Mineralöl, Cetylalkohol, Triglyceride).

In der Creme liegen die asymmetrische lamellare Phospholipid-Aggregate unbeeinflußt durch die Begleitstoffe vor.

### Beispiel 2

18 g lyophilisiertes Phospholipid der Zusammensetzung [60 % PC, 20 % PE (Phosphatidylethanolamin)] werden in 90 ml sterilisiertem Wasser gelöst und mit 16 ml unvergälltem Ethanol versetzt. Mit einem mechanischen hochtourigen Rührer (Ultra-Turrax, 15 000 U/min) wird die Dispersion gerührt und dabei nacheinander Perfluordecalin (CST 22 °C) dem auf 20 °C temperierten Rührbehälter zugesetzt. Die Rohdispersion wird in einem Hochdruckhomogenisator vom Typ Manton Gaulin bei 500 atm im Inertgasstrom homogenisiert. Mit Beginn des vorletzten Durchlaufs werden der Dispersion α-Tocopherolacetat zur Vermeidung von Autoxidationsprozessen und als Fänger für freie Radikale zu 0,1 % zugesetzt.

Die mit dem Photonenkorrelationsspektrometer N-4 MD (Coultronics) durchgeführten Messungen bestätigen das Vorliegen einer unimodalen Teilchengrößenverteilung und einen mittleren Teilchendurchmesser von 128 nm. Die asymmetrischen lamellaren Phospholipid-Aggregate liegen in konzentrische angeordneten, ungeradzahligen Schichten vor, wie aus Kryo-elektronenmikroskopischen Aufnahmen anschaulich zu erkennen ist. Elektronenmikroskopische Untersuchungen bei Anwendung des "negative staining" sind in Übereinstimmung damit. Nach ³¹P-NMR-Untersuchungen liegen die asymmetrischen lamellaren Aggregate im unilamellaren Zustand vor mit einem Zeta-Potential von minus 76 mV.
Die Zusammensetzung der Dispersion beträgt
48 % w/v Perfluordecalin
13 % Phospholipide
9 % Ethanol.

### Beispiel 3

80 g n-F-Hexan, das im Gemisch mit seinen perfluorierten Isomeren vorliegt (CST 20 °C) wurden mit 9,5 Gramm Eigelb 3-sn Phosphatidylcholin in 47 ml deionisiertem und sterilisiertem Wasser unter Inertgasbedingungen bei Zusatz von 0,2 % dl-Alpha-Tocopherol zu einer Rohdispersion mechanisch voremulgiert. Die Rohemulsion wurde im Druckhomogenisator bei Drücken von 500 atm bei einem geeigneten Temperaturregime und unter Kontrolle der Teilchengrößen homogenisiert. Die erhaltene Dispersion weist eine mittlere Viskosität und einen Teilchendurchmesser von 294 nm auf. Nach Zusatz von 8 ml Propylenglycol wurde im Langzeitversuch Stabilität und Keimfreiheit (Keimzahl kleiner als 100 K/g) bei Raumtemperatur beobachtet. Eine Verdünnung, z.B. bei der Herstellung von Lotionen, ist ohne eine Änderung wichtiger kolloidchemischer Parameter problemlos möglich.

Untersuchungen der Dispersion mit dem Lichtmikroskop im polarisierten Licht zeigen das Vorliegen eines isotropen Einphasensystems an, in dem flüssig-kristalline Strukturen nicht existent sind.

### Beispiel 4

### In vivo Nachweis der Liposomen-Penetration

Eine frisch isolierte physiologisch intakte Haut wurde mit ihrer Innenseite auf einen O₂-Sensor (Clark-Elektrode) fixiert und die Epidermis mit einer O₂-transportierenden Dispersion mit asymmetrischen lamellaren Aggregaten benetzt. Unter diesen Bedingungen zeigt die Elektrode keinen O₂-Partialdruck an. Nach 57 Minuten Penetrationsdauer hatten die Aggregate den dermalen Hautabschnitt im Meßbereich der Elektrode erreicht. Der O₂-Partialdruck stieg auf einen Wert von 159 mm Hg an. Die Penetrationsgeschwindigkeit in der Haut ist abhängig von Art und Größe der Aggregate.

### Beispiele 5 bis 19

Die folgenden Beispiele beschreiben kosmetische Formulierungen für spezielle Anwendungen. Die darin enthaltenen Angaben in Prozent sind Gewichtsprozente.

### Beispiel 5 Emulsion (Körperlotion)

| | |
|---|---|
| Polyacrylsäure | 0,30 % |
| TEA | 0,30 % |
| p-Methylhydroxybenzoat | 0,20 % |
| p-Propylhydroxybenzoat | 0,10 % |
| Imidazolidinylharnstoff | 0,20 % |
| Na-EDTA | 0,06 % |
| Cetyl/Stearylalkohol | 1,00 % |
| Stearinsäure | 1,00 % |
| Isopropylmyristinat/-palmitat | 3,00 % |
| Paraffinum subl. | 4,00 % |
| Jojoba-Öl | 2,00 % |
| asymmetrische lamellare Phospholipid-Aggregate | 10,00 % |
| Parfümöl | 1,00 % |
| demineralisiertes Wasser | q.s. |

### Beispiel 6 Emulsion (Creme)

| | |
|---|---|
| Polyacrylsäure | 0,30 % |
| Propylenglycol | 5,00 % |
| TEA | 0,30 % |
| Emulgator 1 | 6,00 % |
| Emulgator 2 | 4,50 % |
| Aloe vera | 2,00 % |
| Reisschalenöl | 1,50 % |
| Cetyl/Stearylalkohol | 1,00 % |
| Jojoba-Öl | 1,50 % |
| p-Methylhydroxybenzoat | 0,20 % |
| p-Propylhydroxybenzoat | 0,10 % |
| Imidazolidinylharnstoff | 0,20 % |
| asymmetrische lamellare Phospholipid-Aggregate | 50,00 % |
| Parfümöl | 1,00 % |
| demineralisiertes Wasser | q.s. |

### Beispiel 7 Emulsion (Reinigungsemulsion)

| | |
|---|---|
| Polyacrylsäure | 0,10 % |
| Propylenglycol | 3,00 % |
| TEA | 0,10 % |
| Emulgator 1 | 5,00 % |
| Emulgator 2 | 2,50 % |
| Linalol-Öl | 1,30 % |
| Avocado-Öl | 2,00 % |
| Jojoba-Öl | 1,50 % |
| p-Methylhydroxybenzoat | 0,20 % |
| p-Propylhydroxybenzoat | 0,10 % |
| Imidazolidinylharnstoff | 0,20 % |
| asymmetrische lamellare Phospholipid-Aggregate | 0,10 % |
| Parfümöl | 0,25 % |
| demineralisiertes Wasser | q.s. |

### Beispiel 8 Emulsion (Maske)

| | |
|---|---|
| Polyacrylasäure | 0,30 % |
| Emulgator 1 | 5,00 % |
| Emulgator 2 | 6,00 % |
| TEA | 0,30 % |
| Aloe vera | 1,50 % |
| Jojoba-Öl | 1,50 % |
| p-Methylhydroxybenzoat | 0,20 % |
| p-Propylhydroxybenzoat | 0,10 % |
| Imidazolidinylharnstoff | 0,20 % |
| asymmetrische lamellare Phospholipid-Aggregate | 40,00 % |
| Parfümöl | 0,50 % |
| demineralisiertes Wasser | qs |

### Beispiel 9 Gel (Gelmaske)

| | |
|---|---|
| Polyacrylsäure | 1,30 % |
| Hydroxyethylcellulose | 0,20 % |
| Propylenglycol | 10,00 % |
| asymmetrische lamellare Phospholipid-Aggregate | 40,00 % |
| TEA | 0,10 % |
| p-Methylhydroxybenzoat | 0,20 % |
| Imidazolidinylharnstoff | 0,30 % |
| Parfümöl | 0,50 % |
| demineralisiertes Wasser | qs |

### Beispiel 10 Sonnenschutzmittel

| | |
|---|---|
| Emulgatorensystem bestehend aus asymmetrischen lamellaren Phospholipid-Aggregaten,Stabilisatoren, Polyglycerinester,Polyoxyethylenester, Isopropylpalmitat | 34,00 % |
| Glycerin | 5,00 % |
| MgSO₄.7H₂O | 0,50 % |
| UV-Filter 1 | 3,00 % |
| UV-Filter 2 | 3,00 % |
| p-Methylhydroxybenzoat | 0,20 % |
| p-Propylhydroxybenzoat | 0,10 % |
| Imidazolidinylharnstoff | 0,30 % |
| Parfümöl | 1,00 % |
| demineralisiertes Wasser | qs |

### Beispiel 11 Shampoo

| | |
|---|---|
| Natriumlaurylethersulfat | 35,00 % |
| Fettsäureamidoalkylbetain | 10,00 % |
| Perglanzkonzentrat | 5,00 % |
| Alkylamidosulfosuccinat | 5,00 % |
| asymmetrische lamellare Phospholipid-Aggregate | 7,50 % |
| Luviquat | 1,00 % |
| Eiweißhydrolysat | 1,00 % |
| Konservierungsmittel | 0,40 % |
| Citronensäure | 0,05 % |
| Parfüm | 0,50 % |
| Steinsalz | 0,50 % |
| demineralisiertes Wasser | qs |

### Beispiel 12 Duschbad

| | |
|---|---|
| Natriumlaurylethersulfat | 45,00 % |
| Fettsäureamidoalkylbetain | 10,00 % |
| Perglanzkonzentrat | 5,00 % |
| asymmetrische lamellare Phospholipid-Aggregate | 12,00 % |
| Citronensäure | 0,05 % |
| Konservierungsmittel | 0,40 % |
| Parfüm | 1,50 % |
| Steinsalz | 1,50 % |
| demineralisiertes Wasser | qs |

### Beispiel 13 Haarkur

| | |
|---|---|
| Polyacrylsäure | 0,50 % |
| Chelaplex | 0,006 % |
| TEA | 0,50 % |
| Propylenglycol | 6,50 % |
| asymmetrische lamellare Phospholipid-Aggregate | 20,00 % |
| Konservierungsmittel | 0,50 % |
| Parfüm | 1,50 % |
| demineralisiertes Wasser | qs |

### Beispiel 14 Deo-Creme

| | |
|---|---|
| Emulgator 1 | 8,00 % |
| Emulgator 2 | 4,00 % |
| Jojoba-Öl | 5,00 % |
| Aloe vera | 5,00 % |
| Propylenglycol | 6,00 % |
| Menthol | 0,10 % |
| Polyacrylsäure | 0,15 % |
| TEA | 0,13 % |
| Konservierungsmittel | 0,50 % |
| asymmetrische lamellare Phospholipid-Aggregate | 25,00 % |
| Parfüm im Deo-Wirkstoff | 1,50 % |
| demineralisiertes Wasser | qs |

### Beispiel 15 After Shave-Balsam

| | |
|---|---|
| Polyacrylsäure | 0,20 % |
| Chelaplex | 0,006 % |
| TEA | 0,20 % |
| Wachs | 1,00 % |
| Glycerin | 4,00 % |
| Jojoba-Öl | 4,00 % |
| Reisschalenöl | 4,00 % |
| Ethanol | 10,00 % |
| asymmetrische lamellare Phospholipid-Aggregate | 37,00 % |
| Konservierungsmittel | 0,50 % |
| Parfüm | 1,50 % |
| demineralisiertes Wasser | qs |

### Beispiel 16 Make up

| | |
|---|---|
| Emulgatorensystem bestehend aus Polyglycerinester, Paraffinum, Polyoxyethylenester, Isopropylpalmitat, Wachse | 25,00 % |
| Aloe vera | 2,00 % |
| Glycerin | 5,00 % |
| MgSO₄.7H₂O | 0,50 % |
| Konservierungsmittel | 0,50 % |
| asymmetrische lamellare Phospholipid-Aggregate | 37,00 % |
| Farbe 1 | 8,50 % |
| Parfümöl | 1,00 % |
| demineralisiertes Wasser | qs |

### Beispiel 17 Augen Make-up

| | |
|---|---|
| Carbopol | 0,20 % |
| TEA | 0,20 % |
| Sorbit | 10,30 % |
| Konservierungsmittel | 0,50 % |
| Paraffinöl | 2,50 % |
| asymmetrische lamellare Phospholipid-Aggregate | 8,00 % |
| Emulgator | 3,70 % |
| Mineralöl | 2,90 % |
| Ethanol | 5,00 % |
| Farbe | 8,00 % |
| demineralisiertes Wasser | qs |

### Beispiel 18 Lidpuder gepreßt mit Lichtschutzfaktor

| | |
|---|---|
| Talkum | 40,00 % |
| Mg-Carbonat | 1,50 % |
| Mg-Stearat | 2,50 % |
| Kaolin | 2,20 % |
| Farben | 15,80 % |
| Perglanzpigmente | 21,50 % |
| Parfümöl | 1,50 % |
| Seidenprotein | 5,00 % |

| Emulsion als Prozeßvermittler | |
|---|---|
| Emulgator | 4,50 % |
| Siliconöl, flüchtig | 2,50 % |
| asymmetrische lamellare Phospholipid-Aggregate | 2,50 % |
| UV-Filter | 2,00 % |
| Konservierung | 0,30 % |
| demineralisiertes Wasser | qs. |

### Beispiel 19 Make up - Transparentpuder gepreßt mit Lichtschutzfaktor

| | |
|---|---|
| Talkum | 70,50 % |
| Kaolin | 10,00 % |
| Mg-Carbonat | 2,50 % |
| Mg-Stearat | 1,50 % |
| Seidenprotein | 2,50 % |
| Farben | 4,50 % |
| Glanzpigmente | 7,50 % |
| Parfümöl | 1,00 % |

| Emulsion als Prozeßvermittler | |
|---|---|
| Emulgator | 4,50 % |
| Siliconöl flüchtig | 2,50 % |
| asymmetrische lamellare Phospholipid-Aggregate | 2,50 % |
| UV-Filter | 2,00 % |
| Konservierung | 0,30 % |
| demineralisiertes Wasser | qs |

## Patentansprüche

1. Phospholipide und Fluorcarbone enthaltendes Kosmetikum, dadurch gekennzeichnet, daß es aus
(a) einem für die kosmetische Anwendung geeigneten Träger ; und
(b) asymmetrischen lamellaren Aggregaten aus Phospholipiden, die einen Phosphatidylcholingehalt im Bereich von 30 bis 99 Gew.-% haben und mit Sauerstoff beladenen Fluorcarbonen im Bereich von 0,2 bis 100 % (Gewicht/Volumen);
mit einer Hautpenetrierung in Abhängigkeit von der kritischen Löslichkeitstemperatur der ausgewählten Fluorcarbone oder Fluorcarbongemische besteht.

2. Kosmetikum nach Anspruch 1, dadurch gekennzeichnet, daß die lamellaren Aggregate von deren Fluorcarbon-Kern ausgehend eine asymmetrische, vorzugsweise dreischichtige Struktur aufweisen.

3. Kosmetikum nach Anspruch 1 oder 2, dadurch gekennzeichnet, daß die Fluorcarbone aus der Gruppe ausgewählt sind, die aus aliphatischen geradkettigen und verzweigten Fluoralkanen, mono- oder bicyclischen gegebenenfalls fluoralkylsubstituierten Fluorcycloalkanen, perfluorierten aliphatischen oder bi-cyclischen Aminen, Bis-(perfluoralkyl)-ethenen, Perfluorpolyethern oder deren Gemischen besteht.

4. Kosmetikum nach Anspruch 3, dadurch gekennzeichnet, daß die Fluorcarbone aus der Gruppe ausgewählt sind, die aus Perfluordecalin, F-Butyltetrahydrofuran, Perfluortributylamin, Perfluoroctylbromid, Bis-Fluor(butyl)ethen oder C₆-C₉-Perfluoralkanen besteht.

5. Kosmetikum nach einem der Ansprüche 1 bis 4, dadurch gekennzeichnet, daß der Anteil an Fluorcarbonen im Bereich von 20 bis 100 % Gewicht/Volumen liegt, vorzugsweise im Bereich von 40 bis 100 %, insbesondere im Bereich von 70 bis 100 %.

6. Kosmetikum nach einem der Ansprüche 1 bis 5, dadurch gekennzeichnet, daß die Phospholipide ausgewählt sind aus der Gruppe, bestehend aus natürlichen Phospholipiden wie Sojalecithin und Eilecithin sowie synthetischen Phospholipiden und/oder teilhydrierten Phospholipiden.

7. Kosmetikum nach einem der Ansprüche 1 bis 6, dadurch gekennzeichnet, daß die verwendete Lipidfraktion Phosphatidylcholin in einem Anteil von 70 bis 99 Gew.-% enthält. %.

8. Kosmetikum nach einem der Ansprüche 1 bis 7, dadurch gekennzeichnet, daß neben Phosphatdylcholin Lysolecithine im Konzentrationsbereich von 1 bis 10 Gew.-% vorhanden sind.

9. Kosmetikum nach einem der Ansprüche 1 bis 8, dadurch gekennzeichnet, daß es zur Erreichung einer langsamen Hautpenetrierung Fluorcarbone oder Fluorcarbongemische mit einer höheren kritischen Löslichkeitstemperatur enthält.

10. Verfahren zur Herstellung eines Phospholipide und Fluorcarbone enthaltenden Kosmetikums, dadurch gekennzeichnet, daß Phospholipide mit einem Phosphatidylcholingehalt von 30 bis 99 Gew.-% mit einem mit Sauerstoff beladenen Fluorcarbon oder Fluorcarbongemisch nach einer Voremulgierung bei höheren Umdrehungszahlen und einer nachfolgenden Hochdruckemulgierung in einen für die kosmetische Anwendung geeigneten und mit den asymmetrischen lamellaren Aggregaten nicht wechselwirkenden Träger eingearbeitet werden, wobei der Anteil an Fluorcarbon im Bereich von 0,2 bis 100 % (Gewicht/Volumen) liegt und die Teilchengröße der asymmetrischen lamellaren Aggregate bei 50 bis 3000 nm, insbesondere 50 bis 1000 nm.

11. Verfahren nach Anspruch 10, dadurch gekennzeichnet, daß die Fluorcarbone aus der Gruppe ausgewählt sind, die aus aliphatischen geradkettigen und verzweigten Fluoralkanen, mono- oder bicyclischen gegebenenfalls fluoralkylsubstituierten Fluorcycloalkanen, perfluorierten aliphatischen oder bi-cyclischen Aminen, Bis-(perfluoralkyl)-ethenen, Perfluorpolyethern oder deren Gemischen ausgewählt sind, und vorzugsweise aus der Gruppe ausgewählt sind, die aus Perfluordecalin, F-Butyltetrahydrofuran, Perfluortributylamin, Perfluoroctylbromid, Bis-Fluor(butyl)ethen oder C₆-C₉-Perfluoralkanen besteht.

12. Verfahren nach Anspruch 10 oder 11, dadurch gekennzeichnet, daß der Anteil an Fluorcarbonen im Bereich von 20 bis 100 % Gew./Vol. liegt, vorzugsweise im Bereich von 40 bis 100 %.

13. Verfahren nach Anspruch 10 oder 11, dadurch gekennzeichnet, daß der Anteil der Phospholipide im Kosmetikum im Bereich von 0,9 bis 15 Gew.% liegt, insbesondere im Bereich von 2 bis 9%.

14. Verwendung eines Phospholipid enthaltenden Kosmetikums zur Steuerung der Sauerstoffversorgung der Haut durch Auftragen eines Systems mit einem asymmetrischen lamellaren Sauerstoff-Carrier, enthaltend Phospholipide mit einem Phosphatidylcholingehalt von 30 bis 99 Gew.-% und Fluorcarbone im Bereich von 0,2 bis 100 % Gewicht/Volumen, wobei die Penetration in die Haut über die Carrier-Struktur der Phopholipid-Aggregate und die kritische Löslichkeitstemperatur der Fluorcarbone (in n-Hexan) gesteuert wird, und das System in einem für die kosmetische Anwendung üblichen Träger wie Salben, Cremes, Lotionen, Wässer, alkoholische Auszüge, Pasten, Gele, Puder, Tinkturen verteilt oder gegebenenfalls auf einem Verband oder einem Pflaster oder als Spray vorliegt.

15. Verwendung nach Anspruch 14, dadurch gekennzeichnet, daß der Gehalt an Phosphatidylcholin in der eingesetzten Lipidfraktion im Bereich von 70 bis 90 % liegt.

## Claims

1. Phospholipid- and fluorcarbon-containing cosmetic, characterised by
(i) a carrier suitable for cosmetic use;
(ii) asymmetric lamellar aggregates, consisting of phospholipids with a content of phosphatidylcholine in the range from 30 to 99 % by weigth and oxygen-laden fluorocarbons, the amount of fluorocarbons being in the range from 0.2 to 100 % weight/volume;
the penetration into the skin being controlled by means of the critical solubility temperature of the selected fluorocarbons or mixture of fluorcarbons.

2. Cosmetic according to Claim 1, characterised in that the lamellar aggregates have an asymmetric, preferably 3-layer structure, originating from their fluorocarbon core.

3. Cosmetic according to Claim 1 or 2, characterised in that the fluorocarbons are selected from the group which consists of aliphatic straight-chain and branched fluoroalkanes, mono- or bicyclic, optionally fluoroalkyl-substituted, fluorocycloalkanes, perfluorinated aliphatic or bicyclic amines, bis(perfluoroalkyl)ethenes, perfluoropolyethers and mixtures thereof.

4. Cosmetic according to Claim 3, characterised in that the fluorocarbons are selected from the group which consists of perfluorodecalin, F-butyltetrahydrofuran, perfluorotributylamine, perfluorooctyl bromide, bis-fluoro(butyl)ethene and C₆-C₉-perfluoroalkanes.

5. Cosmetic according to one of Claims 1 to 4, characterised in that the amount of fluorocarbons is in the range from 20 to 100 % weight/volume, preferably in the range from 40 to 100 %, in particular in the range from 70 to 100 %.

6. Cosmetic according to one of Claims 1 to 5, characterised in that the phospholipids are selected from the group consisting of natural phospholipids such as soya lecithin and egg lecithin and also synthetic phospholipids and/or partially hydrogenated phospholipids.

7. Cosmetic according to one of Claims 1 to 6, characterised in that the lipid fraction used contains phosphatidylcholine in an amount from 70 to 99 %.

8. Cosmetic according to one of Claims 1 to 7, characterised in that, in addition to phosphatidylcholine, lysolecithins are present in the concentration range from 1 to 10 % by weight.

9. Cosmetic according to one of Claims 1 to 8, characterised in that, for reaching a slowly skin penetration, it contains fluorcarbons or mixtures of fluorcarbons with a higher critical solubility temperature.

10. Process for the preparation of a phospholipid-containing cosmetic, characterised in that phospholipids with a content of phosphatidylcholine in the range from 30 to 99 % by weigth together with an oxygen-laden fluorocarbon or fluorocarbon mixture are incorporated after a pre-emulsification at realtively high speed of rotation and a subsequent high-pressure emulsification into a carrier suitable for cosmetic use and non-interacting with the asymmetric lamellar aggregates, the amount of fluorocarbon being in the range from 0.2 to 100 % weight/volume, and the asymmetric lamellar aggregates having a particle size from 50 to 3000 nm, in particular 50 to 1000 nm.

11. Process according to Claim 10, characterised in that the fluorocarbons are selected from the group which consists of aliphatic straight-chain and branched fluoroalkanes, mono- or bicyclic, optionally fluoroalkyl- substituted, fluorocycloalkanes, perfluorinated aliphatic or bicyclic amines, bis(perfluoroalkyl)ethenes, perfluoropolyethers and mixtures thereof, and are preferably selected from the group which consists of perfluorodecalin, F-butyltetrahydrofuran, perfluorotributylamine, perfluorooctyl bromide, bis-fluoro(butyl)ethene and C₆-C₉-perfluoroalkanes.

12. Process according to Claim 10 or 11, characterised in that the amount of fluorocarbons is in the range from 20 to 100 % wt./vol., preferably in the range from 40 to 100 %.

13. Process according to Claim 10 or 11, characterised in that the amount of phospholipids in the cosmetic is in the range from 0.9 to 15 % by weight, in particular in the range from 2 to 9 %.

14. Use of a phospholipid-containing cosmetic for control of the oxygen supply to the skin by applying a system containing an asymmetric lamellar oxygen carrier, containing phospholipids with a content of phosphatidylcholine in the range from 30 to 99 % by weigth and fluorocarbons in the range from 0.2 to 100 % weight/volume, the penetration into the skin being controlled by means of the carrier structure of the phospholipid aggregates and the critical solubility temperature of the fluorocarbons (in n-hexane), and the system being distributed in a carrier which is customary for cosmetic use, such as ointments, creams, lotions, waters, alcoholic extracts, pastes, gels, powders or tinctures, or optionally present on a dressing or a plaster or as a spray.

15. Use according to Claim 14, characterised in that the content of phosphatidylcholine in the lipid fraction employed is in the range from 70 to 90 %.

## Revendications

1. Cosmétique contenant des phospholipides et des fluorocarbones, caractérisé en ce qu'il consiste en :
a) un excipient ou support convenant bien pour une utilisation cosmétique, et
b) des agrégats lamellaires asymétriques de phospholipides, ayant une teneur en phosphatidyl choline se situant entre 30 et 99 % en poids et de fluorocarbones comportant de l'oxygène et présents en une proportion comprise entre 0,2 et 100 % (en poids/volume);
ayant une pénétration dans la peau qui dépend de la température de solubilité critique des fluorocarbones choisis ou des mélanges de fluorocarbones choisis.

2. Cosmétique selon la revendication 1, caractérisé en ce que les agrégats lamellaires dont dérive le noyau fluorocarboné ont une structure asymétrique, présentant avantageusement trois couches.

3. Cosmétique selon la revendication 1 ou 2, caractérisé en ce que les fluorocarbones sont choisis dans l'ensemble formé par des fluoroalcanes aliphatiques linéaires et ramifiés des fluoro- cycloalcanes monocycliques ou bicycliques éventuellement substitués par un ou des groupes fluoroalkyles, des amines perfluorées, aliphatiques ou bicycliques, des bis-(perfluoroalkyl)-éthènes, des polyéthers perfluorés ou leurs mélanges.

4. Cosmétique selon la revendication 3, caractérisé en ce que les fluorocarbones sont choisis dans l'ensemble consistant en de la perfluorédécaline, du F-butyltétrahydrofuranne, de la perfluorotributyl amine, du bromure de perfluoro-octyle, du bis-fluoro(butyl)-éthène ou des perfluoro-alcanes en C₆ à C₉.

5. Cosmétique selon l'une des revendications 1 à 4, caractérisé en ce que la proportion des fluorocarbones se situe entre 20 et 100 % en poids/volume, avantageusement entre 40 et 100 %, notamment entre 70 et 100 %.

6. Cosmétique selon l'une des revendications 1 à 5, caractérisé en ce que les phospholipides sont choisis dans l'ensemble consistant en des phospholipides naturels comme de la lécithine de soja et de la lécithine d'oeuf ainsi qu'en des phospholipides synthétiques et/ou des phospholipides partiellement hydrogénés.

7. Cosmétique selon l'une des revendications 1 à 6, caractérisé en ce que la fraction lipidique utilisée contient de la phosphatidyl choline présente en une proportion de 70 à 99 % en poids.

8. Cosmétique selon l'une des revendications 1 à 7, caractérisé en ce qu'en plus de la phosphatidyl choline des lysolécithines sont présentes en une concentration comprise entre 1 et 10 % en poids.

9. Cosmétique selon l'une des revendications 1 à 8, caractérisé en ce que pour parvenir à une pénétration lente dans la peau, ce cosmétique contient des fluorocarbones ou des mélanges de fluorocarbones ayant une température de solubilité critique élevée.

10. Procédé de préparation d'un cosmétique contenant des phospholipides et des fluorocarbones, procédé caractérisé en ce qu'on incorpore des phospholipides ayant une teneur de 30 à 99 % en poids en de la phosphatidyl choline, avec un fluorocarbone ou un mélange de fluorocarbones comportant de l'oxygène, après une émulsification préalable, à des vitesses élevées de rotation et avec une émulsification subséquente sous pression élevée dans un support convenant pour une application cosmétique et n'ayant pas d'action d'échange avec les agrégats lamellaires asymétriques, la proportion du fluorocarbone se situant entre 0,2 et 100 % (en poids/volume) et la grosseur des particules des agrégats lamellaires asymétriques se situant entre 50 et 3000 nm, notamment entre 50 et 1000 nm.

11. Procédé selon la revendication 10, caractérisé en ce que les fluorocarbones sont choisis dans l'ensemble consistant en des fluoroalcanes aliphatiques, linéaires et ramifiés, des fluorocycloalcanes monocycliques ou bicycliques comportant éventuellement un ou des substituants fluoroalkyles, des amines perfluorées, aliphatiques ou bicycliques, des bis-(perfluoro-alkyl)-éthènes, des polyéthers perfluorés ou leurs mélanges, et avantageusement dans l'ensemble consistant en de la perfluorodécaline, du F-butyltétrahydrofuranne, de la perfluorotributyl amine, du bromure de perfluoro-octyle, du bis-fluoro(butyle)-éthène ou des perfluoroalcanes en C₆ à C₉.

12. Procédé selon la revendication 10 ou 11, caractérisé en ce que la proportion des fluorocarbones se situe entre 20 et 100 % en poids/volume, avantageusement entre 40 et 100 %.

13. Procédé selon la revendication 10 ou 11, caractérisé en ce que la proportion des phospholipides dans le cosmétique se situe entre 0,9 et 15 % en poids, notamment entre 2 et 9 %.

14. Utilisation d'un cosmétique contenant du phospholipide pour réguler l'apport en oxygène pour la peau par l'application d'un système comportant un excipient ou support d'oxygène, lamellaire et asymétrique, contenant des phospholipides ayant une teneur en de la phosphatidyl choline de 30 à 99 % en poids et une teneur en des fluorocarbones de 0,2 à 100 % en poids/volume, la pénétration dans la peau étant régie par la structure du support des agrégats de phospholipides et par la température de solubilité critique. des fluorocarbones (dans du n-hexane), et le système étant réparti dans le support usuel pour l'application cosmétique comme des pommades ou baumes, des crèmes, des potions, des eaux, des extraits alcooliques, des pâtes, des gels, de la poudre, des teintures ou étant éventuellement présent sur un bandage ou un emplâtre ou étant présent sous forme de produit à projeter par nébulisation ou atomisation ("spray").

15. Utilisation selon la revendication 14, caractérisée en ce que la teneur en phosphatidyl choline dans la fraction lipidique utilisée se situe entre 70 et 90 %.
